# EUROPEAN PATENT APPLICATION

(11) **EP 4 365 617 A1**
(43) Date of publication of application: **08.05.2024**
(21) Application number: 22205480.1
(22) Date of filing: 04.11.2022
(51) Int. Cl.: G01R 33/54, G01R 33/561, G01R 33/36, G01R 33/48, G01R 33/565

(54) **TRANSMISSION OF K-SPACE DATA FOR REMOTE RECONSTRUCTION OF MAGNETIC RESONANCE IMAGES**

(71) Applicant: Koninklijke Philips N.V., 5656 AG Eindhoven (NL)
(72) Inventor: CABALLERO, Jose Maria, Eindhoven (NL); PEZZOTTI, Nicola, Eindhoven (NL); GOEDICKE, Andreas Georg, 5656AG Eindhoven (NL); BERGNER, Frank, 5656AG Eindhoven (NL); GRASS, Michael, Eindhoven (NL); SCHNELLBÄCHER, Nikolas David, Eindhoven (NL)
(74) Representative: Philips Intellectual Property & Standards

(57) **Abstract**

A medical system (100) comprising a local memory (138) storing local machine executable instructions (140) and pulse sequence commands (142), a magnetic resonance imaging system (103), and a local computational system (132). The execution of the local machine executable instructions causes the local computational system to transmit (200) metadata descriptive of the magnetic resonance imaging protocol to a remote computational system (132') via a network connection (137). The execution of the local machine executable instructions causes the computational system to repeatedly: control (202) the magnetic resonance imaging system with the pulse sequence commands to acquire one (146) of the series of discrete k-space acquisitions; construct (204) a compressed k-space acquisition (148) by compressing the one of the series of k-space acquisitions using a compression module; and transmit (206) the compressed k-space acquisition to the remote computational system via the network connection.

## Description

### FIELD OF THE INVENTION

The invention relates to magnetic resonance imaging, in particular to the reconstruction of magnetic resonance images.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. A combination of radio-frequency signals and magnetic gradients are used to encode the nuclear spins. The nuclear spins will emit radio- frequency signal in response to this encoding. These radio-frequency signals may be sampled and stored digitally. These samples are in samples in k-space and are referred to herein as k-space data. The k-space data may be Fourier transformed into a magnetic resonance image.

United States patent application publication US2021003651A1 discloses a medical data processing apparatus that includes processing circuitry. The processing circuitry acquires first data pieces obtained by sparse sampling. The processing circuitry generates first compressed data pieces lower in number than the first data pieces by multiplying the first data pieces by each of sets of weight coefficients and adding each of the multiplied first data pieces. The processing circuitry performs first processing of outputting second compressed data pieces by applying a trained model to the first compressed data pieces, the trained model being trained by receiving first compressed data pieces based on sparse sampling and outputting at least one of second compressed data pieces based on full sampling.

### SUMMARY OF THE INVENTION

The invention provides for a medical system, a computer program, and a method in the independent claims. Embodiments are given in the dependent claims.

The amount of data that may be acquired during a magnetic resonance imaging scan may be quite large, the raw data size of the acquired k-space data may be in the hundreds of megabytes or even tens of gigabytes. Once this measured k-space data has been acquired different methods may be used to reconstruct a magnetic resonance image, depending upon the magnetic resonance imaging protocol. Some of these reconstruction methods may be computationally intensive, especially methods that rely on deep learning or on optimization processes to perform the reconstruction. There is therefore interest in performing image reconstruction on remote or cloud-based platforms where computing power can be provided on demand.

A difficulty is that transferring large amount of data to remote systems can result in a delay in receiving the reconstructed magnetic resonance image. Embodiments may provide a means of accelerating this. For example, the measured k-space data is acquired as a series of discrete k-space acquisitions. As a discrete acquisition of k-space data is acquired it is compressed and then transmitted to a remote computational system where the k-space data is decompressed and then used to reconstruct a magnetic resonance image. This may have several advantages. One potential advantage is that the measured k-space data reaches the remote computational system more rapidly and may reduce or alleviate data bottle necks. Another potential advantage is that for some reconstruction techniques the partial data, such as bladed of PROPELLER k-space data or self-navigation data may be used immediately to perform part of the reconstruction.

In one aspect the invention provides for a medical system that comprises a local memory storing local machine-executable instructions and pulse sequence commands. Pulse sequence commands are commands or data which may be converted into commands which are used to control a magnetic resonance imaging system to acquire k-space data. Typically pulse sequence commands are provided in the form of events which happen at different time periods during the acquisition procedure. The medical system further comprises a magnetic resonance imaging system that is configured to acquire measured k-space data that is descriptive of a subject within an imaging zone. The pulse sequence commands are configured to control the magnetic resonance imaging system to acquire the measured k-space data as a series of discrete k-space acquisitions according to a magnetic resonance imaging protocol. The k-space data, which may be used to reconstruct a full image, is the measured k-space data. The series of discrete k-space acquisitions may also be referred to as a series of discrete lines or shots of k-space data.

The medical system further comprises a local computational system. Execution of the local machine-executable instructions causes the local computational system to transmit metadata that is descriptive of the magnetic resonance imaging protocol to a remote computational system via a network connection. Execution of the local machine-executable instructions causes the local computational system to repeatedly control the magnetic resonance imaging system with the pulse sequence commands to acquire one of the series of discrete k-space acquisitions. For example, this could be to acquire one line of k-space data. These meta-data are descriptive of aspects of the acquisition and/or sampling of the measured k-space data. The reconstruction of the image from the measured k-space data may be adapted to the acquisitions and/or sampling aspects of the measured k-space data. To that end , the meta-data are made available at the reconstruction process. This may be implemented by co-transmitting the meta-data with the compressed k-space data, or the meta-data may be made available for the reconstruction process separately transmitted or otherwise made available. Some examples of acquisition and/or sampling aspects may be simple Cartesian k-space sampling, radial or spiral k-space sampling, blade-wise Cartesian sampling where the blades have different orientations in k-space ('multi-vane'). Redundant sampling of the centre region of k-space may be employed for motion correction, or the availability of separate motion data that may be employed for motion correction of the measured k-space data. In the case of parallel imaging with undersampling of k-space, the meta-data may represent that distinct sets of measured k-space data are acquired by a particular receiver antenna (receiver coil). The meta-data may represent aspects of the undersampling pattern, coil sensitivity profiles, or autocalibration data that represent coil sensitivity profiles within the measured k-space data. Such aspects may useful to adapt the reconstruction strategy to the way the k-space data are acquired.

Execution of the local machine-executable instructions causes the local computational system to repeatedly construct a compressed k-space acquisition as one of a series of compressed k-space acquisitions by compressing the one of the series of k-space acquisitions using a compression module. Execution of the local machine-executable instructions further causes the local computational system to repeatedly transmit the compressed k-space acquisition to a remote computational system via the network connection. In these features the magnetic resonance imaging system is controlled to acquire a single discrete k-space acquisition and then compress it into a compressed k-space acquisition. After it has been compressed using a compression module, it is then transmitted to a remote computational system.

Execution of the local machine-executable instructions further causes the local computational system to transmit at least a portion of the series of compressed k-space acquisitions via the network connection before acquisition of the measured k-space data is completed. This embodiment may be beneficial because it provides for an extremely efficient and quick means of transmitting the measured k-space data to a remote computational system. This may for example enable more rapid reconstruction of a magnetic resonance image than would be possible if for example, the measured k-space data were all collected, compressed and then sent to a remote computational system.

In another embodiment the medical system further comprises a remote memory storing remote machine-executable instructions. The medical system further comprises a remote computational system. Execution of the remote machine-executable instructions causes the remote computational system to receive the metadata descriptive of the magnetic resonance imaging protocol via the network connection. Execution of the remote machine-executable instructions further causes remote computational system to repeatedly receive the compressed k-space acquisition via the network connection. Execution of the remote machine-executable instructions further causes remote computational system to repeatedly obtain the one of the series of discrete k-space acquisitions by decompressing the compressed k-space acquisition with a decompression module.

Execution of the machine-executable instructions further causes the remote computational system to reconstruct a magnetic resonance image from at least a portion of the measured k-space data according to the magnetic resonance imaging protocol specified in the metadata. This may take different forms in different examples. In some cases, the system may wait for all of the compressed k-space acquisitions to arrive so that the full measured k-space data can be reconstructed. In this case the system would receive the measured k-space data more rapidly than if it had been collected and then transmitted. In other instances, for example in parallel imaging or where there is self-navigation, such as a propeller magnetic resonance imaging technique, it may be beneficial to have the individual discrete acquisitions earlier. For example, if the discrete acquisitions of k-space are blades of PROPELLER k-space data, then the system can begin motion compensation even before the complete measured k-space data is received. This therefore may enable the reconstruction of the magnetic resonance image more rapidly.

In another embodiment the medical system further comprises a motion detection system for acquiring motion data descriptive of motion of the subject. In some examples the motion detection system may be a sensor system such as a camera, respiration belt or other sensor system. In other examples the motion detection system may work using motion detection by the magnetic resonance imaging system itself. For example, there may be fiducial markers placed on the surface of the subject, there may be self-navigation, or there may be navigation measurements which are used for acquiring the motion data.

Execution of the local machine-executable instructions causes the local computational system to control the motion detection system to acquire the motion data during acquisition of the measured k-space data. Execution of the local machine-executable instructions further causes the local computational system to trigger a motion alert if the motion data indicates subject motion above a predetermined motion threshold. For example, if the subject is moving too often or if the subject repositions his or her body during the acquisition of the measured k-space data, then the trigger may indicate that there may be a need for motion correction.

Execution of the local machine-executable instructions further causes the local computational system to initiate the construction of the compressed k-space acquisition and the transmission of the compressed k-space acquisition to the remote computational system via the network connection if the motion alert is triggered. The metadata comprises a motion compensation reconstruction request for the measured k-space data if the motion alert is triggered.

The motion alert may be indicative of motion of the subject being performed or happening during part of the acquisition of the measured k-space data. The motion alert then requests the remote computational system to perform a reconstruction which performs motion compensation.

As was mentioned above, the motion detection using the motion detection system may not only be detected using an explicit, such as a potential hardware-based motion sensor such as a camera but could also be detected in the acquired raw k-space data or even in images itself. This embodiment is beneficial because motion compensation can be extremely computationally intensive. The ability to detect the motion of the subject and then trigger a remote reconstruction may enable a more powerful computational system to be used to perform the reconstruction.

In another embodiment the compression module is implemented as a neural network. Implementing the compression module as a neural network may be beneficial because neural networks process data extremely quickly and also have very low computational needs. The use of a neural network may therefore enable compression of the discrete k-space acquisitions more rapidly and possibly with less computational requirements and power.

This approach may have one or more of the following benefits:
1. Compression of data in k-space space does not require lossy data pre-processing, hence enabling full utilization of the encoding capacity of the compressive neural network employed.
2. The compression of MR one-dimensional phase encodings (k-space data) allows to independently compress and transmit data from the scanner. This in turn enables multithreading compression and data transmission, increasing throughput.
3. The compression of ID signals (k-space data) allows for the use of smaller encoding networks, reducing encoding and decoding runtimes. In cases where it may be beneficial or necessary to send the compressive neural network weights along with the compressed data, the use of smaller networks would also improve the rate-distortion trade-off.

In another embodiment the compression module is formed from an encoder portion of an autoencoder. The compressed k-space data acquisition is a latent space vector of the autoencoder. The decoder may then be formed from the decoder portion of the autoencoder. This may be beneficial because the k-space data may be transmitted accurately using the latent space vector.

Phase-encoding MR profiles (k-space data) can be arbitrarily defined in 2D or 3D k-space up to certain physiological and scanning parameter constraints. Common choices for sampling trajectories are along Cartesian, radial or spiral coordinates. Regardless of the trajectory design, phase encodings can be linearly projected from 2D or 3D space to a ID space. The ID signal is then processed by an encoding neural network structure, non-linearly projecting it onto a latent ID space of lower dimensionality, thereby achieving compression. The resulting signal can then be transmitted to the receiver end and decoded by a decoding neural network.

The encoding and decoding networks may be trained offline by solving an autoencoding optimization task. Given a training dataset that is assumed to be representative of the data to be compressed, the autoencoder optimizes the neural network parameters such that the output of the autoencoder is as close as possible to the input of the network for all samples in the training dataset. This task can be solved efficiently using stochastic gradient descent or extensions of it, and the metric used to evaluate the faithfulness of the reconstruction can be defined sample-wise in the L2 or L1 sense.

Different embodiments of the invention can alter the encoding-decoding pipeline. Examples of these are:
1. A single autoencoder is applied to all phase encodings independently.
2. Different autoencoders are used for individual phase encodings.
3. A single autoencoder is used for all phase encodings but adapted to individual phase encodings with the transmission of additional information captured during the encoding of each phase encode.

In another embodiment the autoencoder used as the encoder portion and decoder portion is from a vector quantized variational autoencoder (VQ-VAE). Vector quantized variational autoencoders have been shown to be useful for compressing images as was demonstrated in
Razavi, et. al., "Generating diverse high-fidelity images with vq-vae-2." In Advances in neural information processing systems, pp. 14866-14876. 2019. These same techniques may be used to compress the k-space data. Applying a VQ-VAE may be beneficial because the data may be accurately recovered after decompression and the VQ-VAE may be simpler and used fewer computational resources that is necessary for compressing an image.

The VQ-VAE and other autoencoders may be trained to compress data by using previously acquired k-space data as training data for the autoencoder.

In another embodiment the magnetic resonance imaging system comprises a magnetic resonance imaging coil comprising a digitizer circuit for measuring the series of discrete k-space acquisitions. The local computational system comprises the digitizer circuit. The digitizer circuit is configured to construct the compressed k-space acquisition as the one of the series of k-space acquisitions is measured. This embodiment may be beneficial because it may be used to compress the discrete k-space acquisitions as soon as they are acquired. In many magnetic resonance imaging techniques so called parallel imaging is used. This is where there are multiple receive coils being used. If the digitizer circuit for each of these receive coils compresses the discrete k-space acquisitions separately, then the compression and then transmission is performed in a parallel fashion. This may greatly accelerate the transmission of the measured k-space data to the remote computational system.

It should be noted that when a digitizer circuit is used to construct the compressed k-space acquisitions, it may be highly beneficial to use a neural network. This is because the neural networks which may be used for compression such as the variational autoencoder have a very low computational overhead. This means that less computational requirements are necessary for the digitizer circuit as well as reducing the amount of power they use. This may be a concern because the digitizer circuit may be placed in the magnetic resonance imaging system and it is typically not possible to supply them with great amounts of power. This is because of wires going into a magnetic resonance imaging system may pick up some of the radio-frequency and energy used for performing a magnetic resonance imaging protocol. It is therefore highly beneficial to reduce the power use of the digitizer circuit.

In another embodiment execution of the machine-executable instructions further causes the computational system to determine predicted correlations in the discrete acquisitions of k-space from previously acquired k-space data. The metadata comprises the predicted correlations. Execution of the machine-executable instructions further causes the computational system to decorrelate the one of the series of discrete k-space acquisitions using the predicted correlation before compression into the compressed k-space acquisition. Performing this decorrelation essentially reduces the amount of data which needs to be compressed. This may reduce the size of the data which is transmitted to the remote computational system.

In the remote computational system, the receiver may use the metadata about the correlation between the k-space data to reconstruct it once it is decompressed.

In another embodiment the previously acquired k-space data originates from a survey scan of the subject or from a previously compressed one of the series of discrete k-space acquisitions. This may be beneficial because in both cases, such as the survey scan or this previously acquired acquisition, may contain information about how the k-space data can be effectively decorrelated.

In one example, the previously acquired data can be a whole localized scout image, topogram image, or survey image. From these correlations between the points and the k-space data can be derived and transforms can be calculated to decorrelate the data before using the compression techniques. This method is similar to the imaging technique GRAPPA, where actual acquisition is changed to avoid measuring redundant data. For example, samples in the k-space are left out and these can be deduced by neighboring measurements.

In another example, one uses information from previous measurements of the patient such to favorably improve the compression ratio of the next transmitted data part.

In another example, previously transmitted k-space measurements that are already present at the receiver are used to decorrelate the next data that is going to be compressed and transmitted. For this the compression method is additionally fed with the previously transmitted data while compressing the current k-space data which is being compressed. The decoder on the receiving side is behaving in an according or complimentary fashion and using the history of the decompressed chunks so far as to also decompress the current k-space data.

In another aspect, the invention provides for a medical system that comprises a remote memory storing remote machine-executable instructions. The medical system further comprises a remote computational system. Execution of the remote machine-executable instructions causes the computational system to receive metadata which is descriptive of an acquisition of measured k-space data as a series of discrete k-space acquisitions according to a magnetic resonance imaging protocol via a network connection. Execution of the remote machine-executable instructions further causes the remote computational system to repeatedly receive the compressed k-space data acquisitions via the network connection and obtain one of the series of discrete k-space data acquisitions by decompressing the compressed k-space data acquisition with a decompression module. The previously mentioned compression module is complimentary and functions with the decompression module.

Execution of the machine-executable instructions further causes the computational system to reconstruct a magnetic resonance image from at least a portion of the series of discrete k-space acquisitions according to the magnetic resonance imaging protocol specified in the metadata. As was mentioned above, this portion or this medical system forms a receiver which works complimentary with the previously detailed transmitter portion. This receiver portion is able to receive the k-space data more rapidly and thereby either begin a computationally intensive magnetic resonance imaging reconstruction while it is still receiving or it receives the complete measured k-space data more rapidly and is then able to begin the reconstruction sooner.

In another embodiment execution of the remote machine-executable instructions causes the remote computational system to begin reconstruction of the magnetic resonance image before the complete measured k-space data is received. As was mentioned, it is highly beneficial to begin the reconstruction as soon as possible so that the magnetic resonance image is available sooner.

In another embodiment the magnetic resonance imaging protocol is a parallel imaging magnetic resonance imaging protocol. This embodiment may be beneficial because the discrete k-space acquisitions may represent images from individual coils. This enables the remote computational system to begin reconstruction of the magnetic resonance image before the complete measured k-space data is received.

In another embodiment execution of the remote machine-executable instructions causes the remote computational system to begin reconstruction of the magnetic resonance image before the complete measured k-space data is received. The magnetic resonance imaging protocol is a PROPELLER magnetic resonance imaging protocol. In PROPELLER magnetic resonance imaging blades of k-space data are acquired. The discrete k-space acquisition may be a blade of k-space data. The so-called blades are used to reconstruct undersampled blade images which may be used for motion correction. This embodiment may be beneficial because it may enable the motion correction to begin before the complete measured k-space data has arrived.

In another embodiment execution of the remote machine-executable instructions causes the remote computational system to begin reconstruction of the magnetic resonance image before the complete measured k-space data is received. The magnetic resonance imaging protocol is a self-navigation magnetic resonance imaging protocol. This embodiment may be beneficial because the individual discrete acquisitions of k-space data may contain self-navigation data. This embodiment may also enable the motion correction to be calculated before the complete measured k-space data is received.

In another embodiment execution of the remote machine-executable instructions causes the remote computational system to begin reconstruction of the magnetic resonance image after the complete measured k-space data is received. This for example may be useful for extremely computationally intensive magnetic resonance imaging protocols where the reconstruction has a large computational overhead.

In another embodiment the magnetic resonance imaging protocol is a magnetic resonance fingerprinting magnetic resonance imaging protocol. This is one example where there is a large computational overhead and although the system may wait until the completed measured k-space data is received, it however receives the complete measured k-space data faster so it may be beneficial.

In another embodiment the magnetic resonance imaging protocol is a motion compensating magnetic resonance imaging protocol. This may for example be a magnetic resonance imaging protocol where the self-consistency of the motion correction is checked with the originally measured k-space data. This may also have a large computational overhead. So even if the reconstruction does not begin until the complete measured k-space data has been received, it may still be advantageous because the remote computational system is able to begin sooner on this computationally intense reconstruction.

In another aspect the invention provides for a computer program that comprises local machine-executable instructions for execution by a local computational system that is configured to control a magnetic resonance imaging system. The computer program may also comprise pulse sequence commands. The magnetic resonance imaging system is configured to acquire measured k-space data that is descriptive of a subject within an imaging zone. The pulse sequence commands are configured to control the magnetic resonance imaging system to acquire the measured k-space data as a series of discrete k-space acquisitions according to a magnetic resonance imaging protocol.

The execution of the local machine-executable instructions causes the local computational system to transmit metadata that is descriptive of the magnetic resonance imaging protocol to a remote computational system via a network connection. The execution of the local machine-executable instruction causes the local computational system to repeatedly control the magnetic resonance imaging system with the pulse sequence commands to acquire one of the series of discrete k-space acquisitions. The execution of the local machine-executable instructions further causes the local computational system to repeatedly construct a compressed k-space acquisition as one of the series of compressed k-space acquisitions by compressing the one of the series of k-space acquisitions using a compression module. The execution of the local machine-executable instructions further causes the local computational system to repeatedly transmit the compressed k-space acquisition to the remote computational system via the network connection. The execution of the local machine-executable instructions further causes the local computational system to transmit at least a portion of the series of compressed k-space acquisitions via the network connection before acquisition of the measured k-space data is completed.

In another aspect the invention provides for a method of operating a medical system. The medical system comprises a local memory storing machine-executable instructions and pulse sequence commands. The method further comprises a remote memory storing remote machine-executable instructions. The medical system further comprises a magnetic resonance imaging system that is configured to acquire measured k-space data descriptive of a subject within an imaging zone. The pulse sequence commands are configured to control the magnetic resonance imaging system to acquire the measured k-space data as a series of discrete k-space acquisitions according to a magnetic resonance imaging protocol. The medical system further comprises a local computational system. The medical system further comprises a remote computational system.

The execution of the local machine-executable instructions causes the local computational system to transmit metadata that is descriptive of the magnetic resonance imaging protocol to the remote computational system via a network connection.

The execution of the remote machine-executable instructions causes the remote computational system to receive the metadata via the network connection.

The execution of the local machine-executable instructions causes the local computational system to repeatedly control a magnetic resonance imaging system with the pulse sequence commands to acquire one on the series of discrete k-space acquisitions. The execution of the local machine-executable instructions further causes the local computational system to repeatedly construct a compressed k-space acquisition as one of the series of compressed k-space acquisitions by compressing the one of the series of k-space acquisitions using a compression module. The execution of the local machine-executable instructions further causes the local computational system to repeatedly transmit the compressed k-space acquisition to the remote computational system via the network connection.

The execution of the local machine-executable instructions further causes the local computational system to transmit at least a portion of the series of compressed k-space acquisitions via the network connection before acquisition of the measured k-space data is completed.

The execution of the remote machine-executable instructions further causes the remote computational system to repeatedly receive the compressed k-space acquisition via the network connection. The execution of the remote machine-executable instructions further causes the remote computational system to repeatedly obtain one of the series of discrete k-space acquisitions by decompressing the compressed k-space acquisition with a decompression module. The execution of the machine-executable instructions further causes the remote computational system to reconstruct a magnetic resonance image from at least a portion of the series of discrete k-space acquisitions according to the magnetic resonance imaging protocol specified in the metadata.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 shows an example of a radio frequency coil with multiple coil elements;
Fig. 4 illustrates an example of an autoencoder that may be used to compress k-space data;
Fig. 5 illustrates examples of image domain and k-space domain compression; and
Fig. 6 shows a timing diagram for the examples illustrated in Fig. 5.

### DETAILED DESCRIPTION OF EMBODIMENTS

Like numbered elements in these figures are either equivalent elements or perform the same function. Elements which have been discussed previously will not necessarily be discussed in later figures if the function is equivalent.

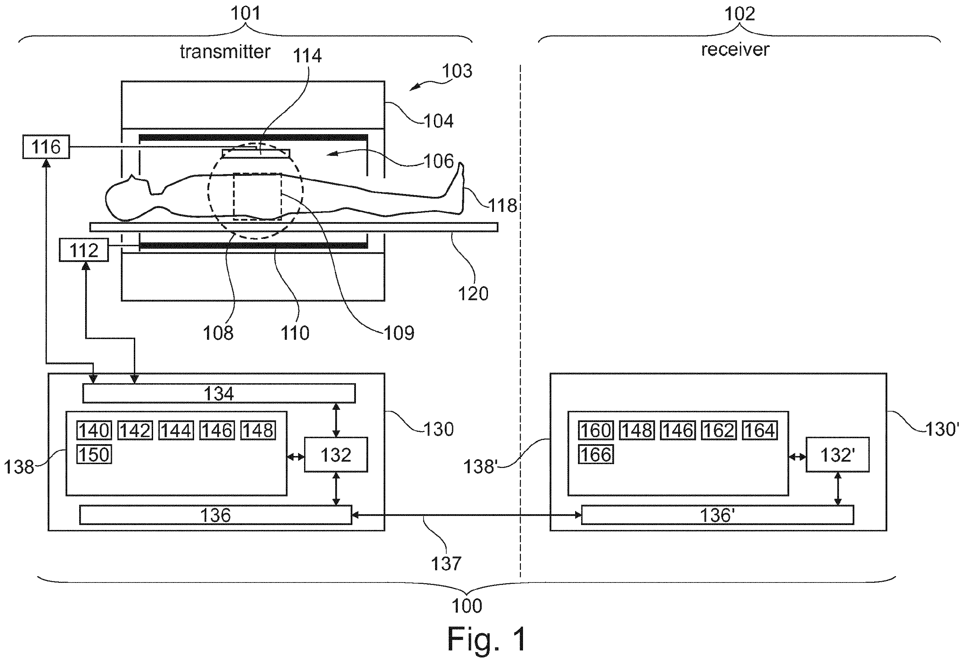

Fig. 1 illustrates an example of a medical system 100 that comprises a transmitter portion 101 and a receiver portion 102. The transmitter portion 101 is shown as being formed from a magnetic resonance imaging system 103 and a local computer 130. The receiver portion is shown as being formed from a remote computer 130'.

The magnetic resonance imaging system 103 comprises a magnet 104. The magnet 104 is a superconducting cylindrical type magnet with a bore 106 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 106 of the cylindrical magnet 104 there is an imaging zone 108 where the magnetic field is strong and uniform enough to perform magnetic resonance imaging. A field of view 109 is shown within the imaging zone 108. The magnetic resonance data that is acquired typically acquired for the field of view 109. A subject 118 is shown as being supported by a subject support 120 such that at least a portion of the subject 118 is within the imaging zone 108 and the predetermined region of interest 109.

Within the bore 106 of the magnet there is also a set of magnetic field gradient coils 110 which is used for acquisition of preliminary magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically, magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone 108. The radio frequency antenna may contain multiple coil elements. The radio frequency antenna may also be referred to as a channel or antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise, the transceiver 116 may also represent a separate transmitter and receivers. The radio-frequency coil 114 may also have multiple receive/transmit elements and the radio frequency transceiver 116 may have multiple receive/transmit channels. For example, if a parallel imaging technique such as SENSE is performed, the radio-frequency coil could 114 will have multiple coil elements.

The transmitter portion 101 is further shown as comprising the local computer 130. The transceiver 116 and the gradient controller 112 are shown as being connected to the hardware interface 134 of the local computer system 130. The local computer 130 is intended to represent one or more computers that are located in the vicinity of the magnetic resonance imaging system 103. The local computer 130 comprises a local computational system 132. The local computational system 132 is intended to represent one or more computational or computing cores. The local computational system 132 is shown as being in communication with a hardware interface 134 that enables the local computational system 132 to communicate with the magnetic resonance imaging system 103 to control it and to receive k-space data. The local computational system 132 is further shown as being in communication with a local network connection 136 and a local memory 138. The local network connection 136 enables the local computer 130 to communicate with the remote computer 130' via a network interface 137.

The local memory 138 is intended to represent various types of memories which are accessible to the local computational system 132. The local memory 138 is shown as storing local machine-executable instructions 140. The local machine-executable instructions 140 enable the local computational system 132 to perform various computational and data handling and image processing tasks. The local memory 138 is further shown as containing pulse sequence commands 142. The pulse sequence commands are commands or data which may be converted into commands which enable the local computational system 132 to control and operate the magnetic resonance imaging system 103 via the hardware interface 134.

The memory 138 is further shown as containing metadata 144 that is descriptive of the pulse sequence commands 142. The metadata is used by the remote computer 130' to determine which magnetic resonance imaging protocol to use when reconstructing a magnetic resonance image. The local memory 138 is further shown as containing one of a series of discrete k-space acquisitions 146 that was acquired when controlling the magnetic resonance imaging system 103 with the pulse sequence commands 142. The memory 138 is further shown as containing a compressed k-space acquisition 148 that was a compression of the one of the series of discrete k-space acquisitions 146 that was compressed with the compression module 150. The compression module 150 is shown as being stored by the local memory 138.

The receiver portion 102 is shown as comprising the remote computer 130'. The remote computer 130' is shown as containing a remote computational system 132'. The remote computer 130' could for example be an implementation of a cloud-based reconstruction service for the magnetic resonance imaging system 103. The remote computational system 132' is shown as being in communication with a remote memory 138' and a remote network interface 136'. The remote network interface 136' is used to form the network connection 137 with the local network interface 136. The network connection 137 could for example be a LAN, an internet connection, or a wireless data communication. The remote memory 138 is intended to represent various types of memories that are accessible to the remote computational system 132'.

The remote memory 138' is shown as containing remote machine-executable instructions 160. These contain commands which enable the remote computational system 132' to perform basic data processing and numerical and image processing tasks. The remote memory 138' is further shown as containing the compressed k-space acquisition that was received via the network connection 137. The remote memory 138' is further shown as containing one of the series of discrete k-space acquisitions 146 that was obtained by decompressing the compressed k-space acquisition 148 with a decompression module 162. The remote memory 138' is further shown as containing the series of discrete k-space acquisitions 164 which in this case is equivalent to the measured k-space data. The memory 138' is further shown as containing a magnetic resonance image 166 that was reconstructed from the series of discrete k-space acquisitions 164 or the measured k-space data.

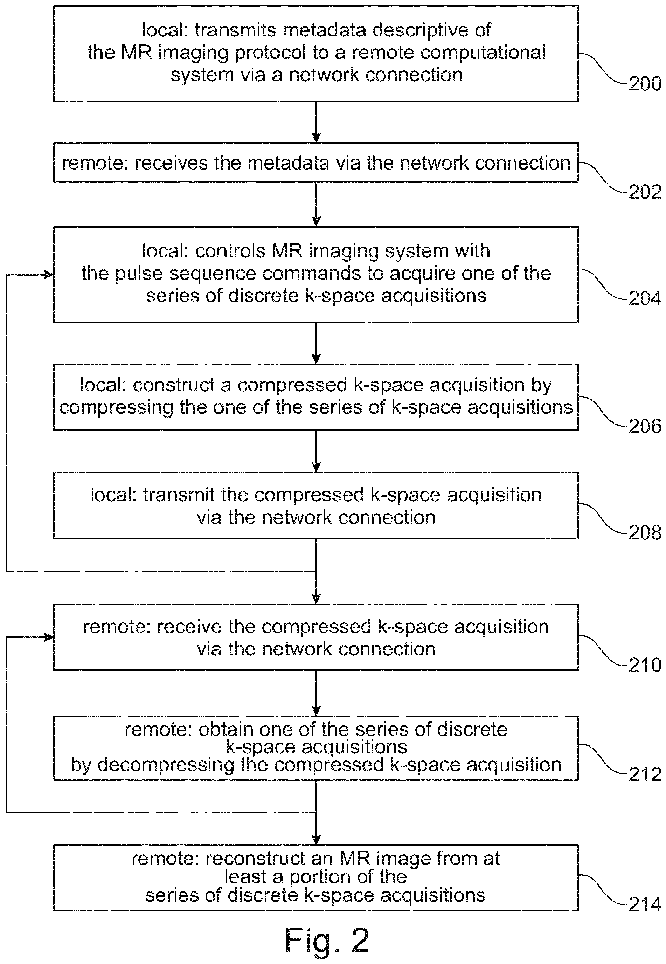

Fig. 2 shows a flowchart which illustrates a method of operating the medical system of Fig. 1. First, in step 200, the local computational system 132 transmits the metadata 144 via the network connection 137 to the remote computational system 132'. Next, in step 202, the remote computational system 132' receives the metadata 144 via the network connection 137. Next, in step 204, the local computational system 132 controls the magnetic resonance imaging system 103 with the pulse sequence commands 142 to cause it to acquire the series of discrete k-space acquisitions 146. The magnetic resonance imaging system does this continuously. Steps 204, 206, and 208 may be performed in parallel as they are able to be performed. Next, in step 206, a compressed k-space acquisition 148 is constructed by compressing the one of the series of discrete k-space acquisitions 146 with the compression module 150. After this compressed k-space acquisition 148 has been constructed, step 208 is performed.

In step 208 the local computational system 132 transmits the compressed k-space acquisition 148 via the network interface 137 to the remote computational system 132'. After step 208 is performed the magnetic resonance imaging system can continuously acquire k-space data. This is indicated by the arrow returning to step 204. While this is happening, the method may also proceed to step 210. Step 210 represents what happens after one compressed k-space acquisition 148 has been transmitted. Next, in step 210, the remote computational system 132' receives the compressed k-space acquisition 148 via the network connection 137. Steps 210 and 212 can be performed in parallel as in after in step 212 the one of a series of discrete k-space acquisitions 148 is obtained by decompressing the compressed k-space acquisition 148 with the decompression module 162.

The method then proceeds back to step 210 and also step 214. The step back to step 210 indicates how the system can be continuously receiving compressed k-space data and decompressing it. In some instances, the system will wait for the complete series of discrete k-space acquisitions 164 or measured k-space data to be received and then step 214 will be performed, which is to reconstruct a magnetic resonance image 166 from at least a portion of the series of discrete k-space ?? data 164. In other examples there may be techniques such as motion compensation or parallel imaging where the remote computational system 132' can begin the reconstruction of the magnetic resonance image 166 before all of the series of discrete k-space acquisitions 164 are received. In this case step 214 may proceed as step 210 and 212 are being continuously performed.

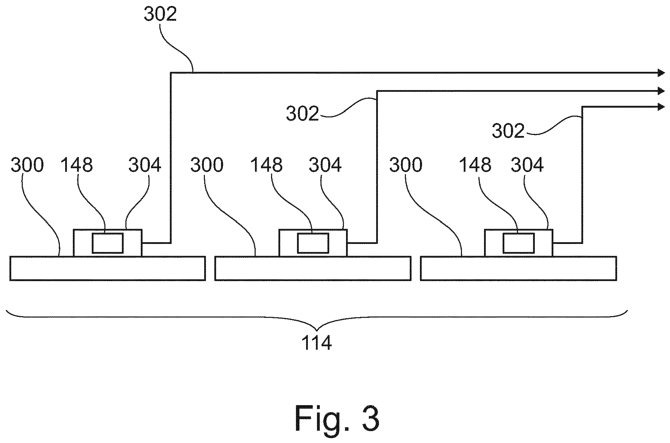

Fig. 3 illustrates one implementation of a radio-frequency coil 114. In this example there are multiple coil elements 300 that independently receive k-space data. The coil elements comprise an optical connection 302 to the hardware interface 134 and also a digitizer 304 or DSP circuitry that is used to measure the k-space data. It can be seen that the digitizers 304 have received and then compressed the measured k-space data into compressed k-space acquisitions 148. These are sent directly via the optical connection 302 to the local computational system 132 that forwards it to the remote computational system 132' via the network connection 137. A possible advantage of the system illustrated in Fig. 3 is that the k-space data is compressed immediately and is done in a parallel fashion. It should be noted that in Fig. 3 one possible implementation of a compression module 150 would be to use a neural network. This may be particularly advantageous because it requires less computational overhead and may reduce the power requirements on the digitizers 304.

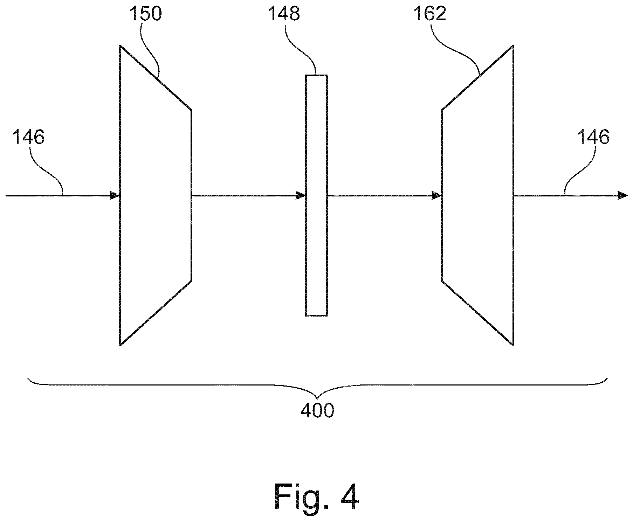

Fig. 4 illustrates an example of how an autoencoder 400 can be used to form a compression module 150 and a decompression module 162. In this example, the compression module 150 is the encoder portion of the autoencoder 400 and the decompression module 162 is the decoder portion of the autoencoder. The encoder portion or the compression module 150 receives the discrete k-space acquisition 146 as input and then outputs a latent space vector or the compressed k-space acquisition 148. The compressed k-space acquisition 148 can be transmitted via the network interface 137 where it is then input into the decompression module 162, which is the decoder portion of the autoencoder 400. In response to receiving the compressed k-space acquisition 148 the decompression module 162 outputs the discrete k-space acquisition 146.

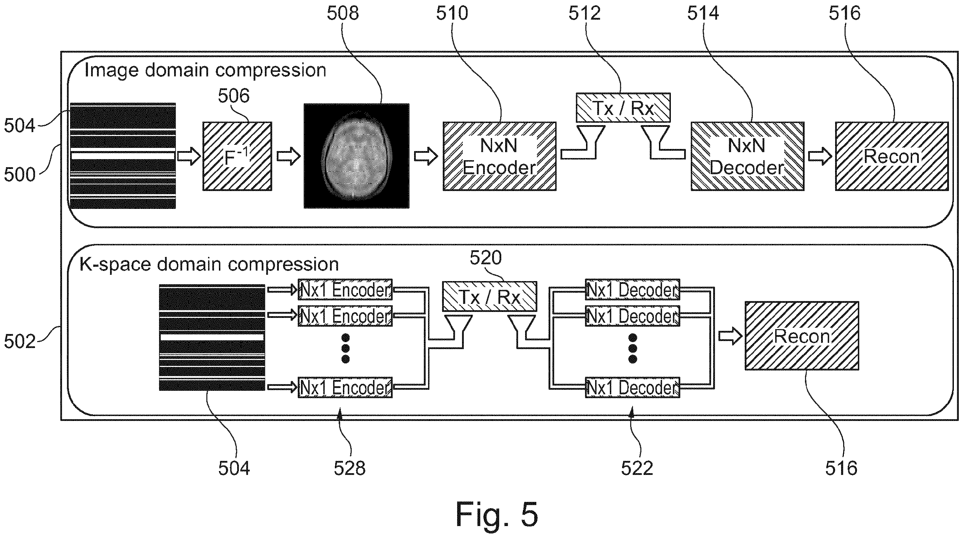

Fig. 5 illustrates a method of image domain compression 500 and a method of k-space domain compression 502 to transmit magnetic resonance imaging data such as k-space data to a remote computing system. Fig. 5 provides a high-level comparison of MR compression in image domain 500 and the proposed framework in k-space domain 502 for a single slice brain scan of dimensions N x N. Compression in image domain requires all phase-encoding profiles to be acquired before pre-processing, domain transform and encoding can be applied. In contrast, encoding individual k-space phase encodings allows to multithread encoding, transmission (Tx / Rx) and decoding of raw data.

The image domain compression 500 starts with the acquisition 504 and proceeds, after all the data has been acquired, to perform a Fourier transform 506 which results in the magnetic resonance image 508. There is then an NxN encoder or a compression 510 which is performed. It is noted that all of the data is acquired before the image 508 is reconstructed. This is then transmitted 512 where it is received by the remote computing system and is put through an NxN decoder 514. The NxN decoder is a decompression algorithm. This is then fed to the reconstruction 516.

The k-space domain compression 502 functions differently. The acquisition 504 takes place but as various bits of k-space data become available they are encoded parallel 518 and then transmitted immediately 520 via the network interface 137. As they are received they are decoded or decompressed in parallel or asynchronously 522. When the data is fully received or partially received it is gone to the reconstruction 516.

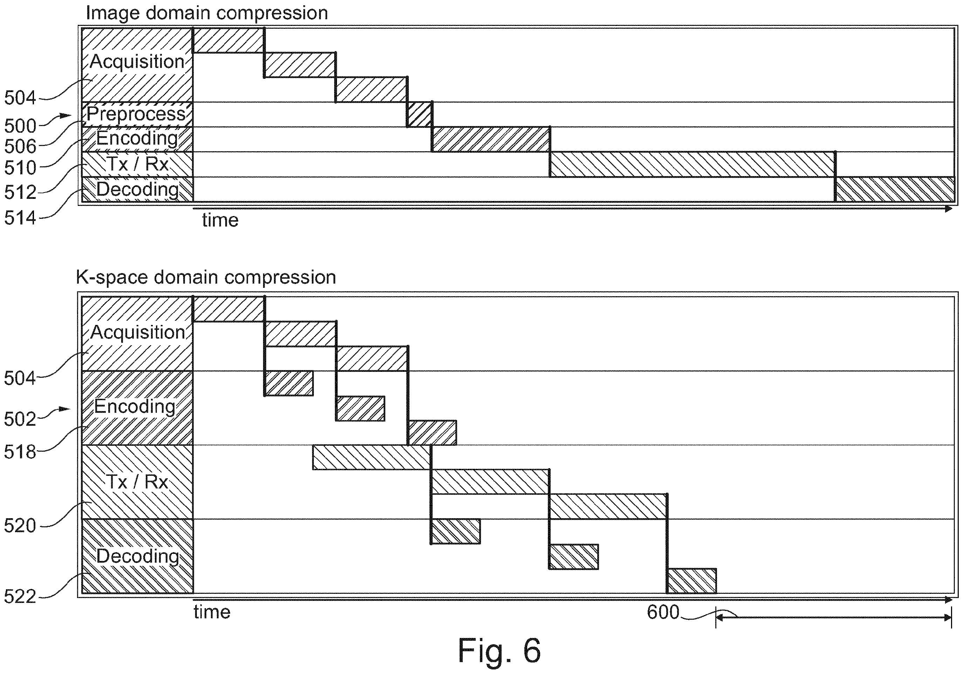

Fig. 6 is used to illustrate the benefit of the k-space domain compression 502 over the image domain compression 500. Fig. 6 shows a threading diagram for encoding, transmission and decoding in image domain 500 and k-space domain 502. Compression in k-space domain enables treating raw data in phase encodings independently. Data can therefore be encoded, transmitted (Tx / Rx) and decoded before acquisition is finished, and a Cloud-based reconstruction could benefit from a higher throughput of data transmission. Fig. 6 shows a timing chart is shown for the image domain compression 500 and the k-space domain compression 502. In the image domain compression 500 the acquisition 504 is performed and after this is completely finished the pre-processing or Fourier transform 506 happens. After this is completed the encoding 510 is performed where it is then transmitted 512 and then decoded 514. Each of these processes are performed serially parallel.

In contrast, the k-space domain compression performs many tasks in parallel or simultaneously. As the acquisition 504 takes place, encoding 518 takes place as various bits of k-space data are available. As the encoding takes place, they are transmitted 520 as soon as they are available. The decoding 522 also takes place partially as the packets of k-space data are still being transmitted. It can be seen that the decoding 522 is completely finished for the k-space domain compression much sooner than the image domain compression 500. This is illustrated by the time savings 600.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 101: transmitter portion
- 102: receiver portion
- 103: magnetic resonance imaging system
- 104: magnet
- 106: bore of magnet
- 108: imaging zone
- 109: field of view
- 110: magnetic field gradient coils
- 112: magnetic field gradient coil power supply
- 114: radio-frequency coil
- 116: transceiver
- 118: subject
- 120: subject support
- 130: local computer
- 130': remote computer
- 132: local computational system
- 132': remote computational system
- 134: local hardware interface
- 136: local network connection
- 136': remote network connection
- 137: network connection
- 138: local memory
- 138': remote memory
- 140: local machine executable instructions
- 142: pulse sequence commands
- 144: metadata
- 146: one of a series of discrete k-space acquisitions
- 148: compressed k-space acquisition
- 150: compression module
- 160: remote machine executable instructions
- 162: decompression module
- 164: series of discrete k-space acquisitions (measured k-space data)
- 166: magnetic resonance image
- 200: local computational system: transmit metadata descriptive of the magnetic resonance imaging protocol to a remote computational system via a network connection
- 202: remote computational system: to receive the metadata descriptive of the acquisition of measured k-space data as the series discrete k-space acquisitions according to the magnetic resonance imaging protocol via the network connection
- 204: local computational system: control a magnetic resonance imaging system with the pulse sequence commands to acquire one of the series of discrete k-space acquisitions
- 206: local computational system: construct a compressed k-space acquisition as one of a series of compressed k-space acquisitions by compressing the one of the series of k-space acquisitions using a compression module
- 208: local computational system: transmit the compressed k-space acquisition to remote computational system via the network connection
- 210: remote computational system: receive the compressed k-space acquisition via the network connection
- 212: remote computational system: obtain one of the series of discrete k-space acquisitions by decompressing the compressed k-space acquisition with a decompression module
- 214: remote computational system: reconstruct a magnetic resonance image from at least a portion of the series of discrete k-space acquisitions
- 300: coil elements
- 302: optical connection
- 304: digitizer (DSP)
- 400: autoencoder
- 500: image domain compression
- 502: k-space domain compression
- 504: acquisition
- 506: fourier transform
- 508: magnetic resonance image
- 510: NxN encoder
- 512: transmission
- 514: NxN decoder
- 516: reconstruction
- 518: parallel encoding
- 520: transmission
- 522: parallel decoding

## Claims

1. A medical system (100) comprising:
- a local memory (38) storing local machine executable instructions (140) and pulse sequence commands (142);
- a magnetic resonance imaging system (103) configured to acquire measured k-space data descriptive of a subject (118) within an imaging zone (108), wherein the pulse sequence commands are configured to control the magnetic resonance imaging system to acquire the measured k-space data as a series of discrete k-space acquisitions (164) according to a magnetic resonance imaging protocol;
- a local computational system (132), wherein execution of the local machine executable instructions causes the local computational system to transmit (200) metadata descriptive of the magnetic resonance imaging protocol to a remote computational system (132') via a network connection (137), wherein execution of the local machine executable instructions causes the computational system to repeatedly:
- control (202) the magnetic resonance imaging system with the pulse sequence commands to acquire one (146) of the series of discrete k-space acquisitions;
- construct (204) a compressed k-space acquisition (148) as one of a series of compressed k-space acquisitions by compressing the one of the series of k-space acquisitions using a compression module (150); and
- transmit (206) the compressed k-space acquisition to the remote computational system via the network connection; and
wherein execution of the local machine executable instructions causes the local computational system to transmit at least a portion of the series of compressed k-space acquisitions via the network connection before acquisition of the measured k-space data is completed.

2. The medical system of claim 1, wherein the medical system further comprises:
- a remote memory (138') storing remote machine executable instructions (160);
- a remote computational system (132'), wherein execution of the remote machine executable instructions causes the remote computational system to receive (202) the metadata descriptive of the magnetic resonance imaging protocol via the network connection, wherein execution of the remote machine executable instructions further causes the remote computational system to repeatedly:
- receive (210) the compressed k-space acquisition via the network connection; and
- obtain (212) the one of the series of discrete k-space acquisitions by decompressing the compressed k-space acquisition with a decompression module; and
wherein execution of the machine executable instructions further causes the remote computational system to reconstruct (214) a magnetic resonance image (166) from at least a portion of the measured k-space data according to the magnetic resonance imaging protocol specified in the metadata.

3. The medical system of claim 1 or 2, wherein the medical system further comprises a motion detection system for acquiring motion data descriptive of motion of the subject, wherein execution of the local machine executable instructions causes the local computational system to:
- control the motion detection system to acquire the motion data during acquisition of the measured k-space data;
- trigger a motion alert if the motion data indicates subject motion above a predetermined motion threshold;
- initiate the construction of the compressed k-space acquisition and the transmission of the compressed k-space acquisition to the remote computational system via the network connection if the motion alert is triggered, wherein the metadata comprises a motion compensation reconstruction request for the measured k-space data if the motion alert is triggered.

4. The medical system of claim 1, 2, or 3, wherein the compression module is implemented as a neural network.

5. The medical system of claim 4, wherein the compression module is formed from an encoder portion of an autoencoder, wherein the compressed k-space acquisition is a latent space vector of the autoencoder.

6. The medical system of any one of the preceding claims, wherein the magnetic resonance imaging system comprises a magnetic resonance imaging coil comprising a digitizer circuit (304) for measuring the series of discrete k-space acquisitions, wherein the local computational system comprises the digitizer circuit, wherein the digitizer circuit is configured to construct the compressed k-space acquisition as the one of the series of k-space acquisitions is measured.

7. The medical system of any one of the preceding claims, wherein execution of the machine executable instructions further causes the computational system to:
- determine predicted correlations in the discrete acquisitions of k-space from previously acquired k-space data, wherein the metadata comprises the predicted correlations;
- decorrelate the one of the series of discrete k-space acquisitions using the predicted correlations before compression into the compressed k-space acquisition;

8. The medical system of claim 7, wherein the previously acquired k-space data originates from a survey scan of the subject or from a previously compressed one of the series of discrete k-space acquisitions.

9. A medical system (100) comprising:
- a remote memory (138') storing remote machine executable instructions (160);
- a remote computational system (132'), wherein execution of the remote machine executable instructions causes the computational system to receive (202) metadata (144) descriptive of an acquisition of measured k-space data as a series discrete k-space acquisitions (164) according to a magnetic resonance imaging protocol via a network connection (137), wherein execution of the remote machine executable instructions further causes the remote computational system to repeatedly:
- receive (210) the compressed k-space acquisition via the network connection; and
- obtain (212) one of the series of discrete k-space acquisitions by decompressing the compressed k-space acquisition with a decompression module; and
wherein execution of the machine executable instructions further causes the computational system to reconstruct (214) a magnetic resonance image (166) from at least a portion of the series of discrete k-space acquisitions according to the magnetic resonance imaging protocol specified in the metadata.

10. The medical system of claim 9, wherein execution of the remote machine executable instructions causes the remote computational system to begin reconstruction of the magnetic resonance image before the complete measured k-space data is received.

11. The medical system of claim 10, wherein any one of the following:
- the magnetic resonance imaging protocol is a parallel imaging magnetic resonance imaging protocol;
- the magnetic resonance imaging protocol is a PROPELLER magnetic resonance imaging protocol; and
- the magnetic resonance imaging protocol is a self-navigation magnetic resonance imaging protocol.

12. The medical system of claim 9, wherein execution of the remote machine executable instructions causes the remote computational system to begin reconstruction of the magnetic resonance image after the complete measured k-space data is received.

13. The medical system of claim 12, wherein any one of the following:
- the magnetic resonance imaging protocol is a magnetic resonance fingerprinting magnetic resonance imaging protocol; and
- the magnetic resonance imaging protocol is a motion compensating magnetic resonance imaging protocol.

14. A computer program comprising local machine executable instructions (140) for execution by a local computational system (132) configured to control a magnetic resonance imaging system (103), wherein the magnetic resonance imaging system is configured to acquire measured k-space data descriptive of a subject within an imaging zone, wherein the pulse sequence commands are configured to control the magnetic resonance imaging system to acquire the measured k-space data as a series of discrete k-space acquisitions (164) according to a magnetic resonance imaging protocol;
wherein execution of the local machine executable instructions causes the local computational system to transmit (200) metadata (144) descriptive of the magnetic resonance imaging protocol to a remote computational system (132') via a network connection (137), wherein execution of the local machine executable instructions causes the local computational system to repeatedly:
- control (204) the magnetic resonance imaging system with the pulse sequence commands to acquire one (146) of the series of discrete k-space acquisitions;
- construct (206) a compressed k-space acquisition (148) as one of a series of compressed k-space acquisitions by compressing the one of the series of k-space acquisitions using a compression module; and
- transmit (208) the compressed k-space acquisition to the remote computational system via the network connection; and
- wherein execution of the local machine executable instructions causes the local computational system to transmit at least a portion of the series of compressed k-space acquisitions via the network connection before acquisition of the measured k-space data is completed.

15. A method of operating a medical system (100), wherein the medical system comprises:
- a local memory (138) storing local machine executable instructions (140) and pulse sequence commands (142);
- a remote memory (138') storing remote machine executable instructions (160);
- a magnetic resonance imaging system (103) configured to acquire measured k-space data descriptive of a subject (118) within an imaging zone (108), wherein the pulse sequence commands are configured to control the magnetic resonance imaging system to acquire the measured k-space data as a series of discrete k-space acquisitions (164) according to a magnetic resonance imaging protocol;
- a local computational system (138); and
- a remote computational system (138');
wherein execution of the local machine executable instructions causes the local computational system to transmit (200) metadata (144) descriptive of the magnetic resonance imaging protocol to the remote computational system via a network connection (137);
wherein execution of the remote machine executable instructions causes the remote computational system to receive (202) the metadata via the network connection;
wherein execution of the local machine executable instructions causes the local computational system to repeatedly:
- control (204) the magnetic resonance imaging system with the pulse sequence commands to acquire one of the series of discrete k-space acquisitions;
- construct (206) a compressed k-space acquisition (148) as one of a series of compressed k-space acquisitions by compressing the one of the series of k-space acquisitions using a compression module; and
- transmit (208) the compressed k-space acquisition to the remote computational system via the network connection;
wherein execution of the local machine executable instructions causes the local computational system to transmit at least a portion of the series of compressed k-space acquisitions via the network connection before acquisition of the measured k-space data is completed;
wherein execution of the remote machine executable instructions further causes the remote computational system to repeatedly:
- receive (210) the compressed k-space acquisition via the network connection; and
- obtain (212) one of the series of discrete k-space acquisitions by decompressing the compressed k-space acquisition with a decompression module; and
wherein execution of the machine executable instructions further causes the remote computational system to reconstruct (214) a magnetic resonance image (166) from at least a portion of the series of discrete k-space acquisitions according to the magnetic resonance imaging protocol specified in the metadata.
